# EUROPÄISCHE PATENTANMELDUNG

(11) **EP 2 774 614 A1**
(43) Veröffentlichungstag der Anmeldung: **10.09.2014**
(21) Anmeldenummer: 14164080.5
(22) Anmeldetag: 22.10.2008
(51) Int. Cl.: A61K 31/565, A61K 31/57, A61P 15/16

(54) **Verwendung eines Gestagens in Kombination mit einem Estrogen und einem oder mehreren pharmazeutisch annehmbaren Hilfsstoffen/ Trägern zur laktosefreien oralen Kontrazeption**

(30) Priorität: 05.11.2007 EP 07021465
(62) Teilanmeldung aus: 08847408.5
(71) Anmelder: Bayer Pharma Aktiengesellschaft, 13353 Berlin (DE)
(72) Erfinder: Claussen, Claus, 07743 Jena (DE); Fricke, Sabine, 07749 Jena (DE); Pfeifer, Manuela, 07745 Jena (DE); Ladwig, Ralf, 07747 Jena (DE); Buerglein, Beate, 07743 Jena (DE)
(74) Vertreter: BIP Patents

(57) **Zusammenfassung**

Gestagene, bevorzugt Dienogest, Chlormadinonacetat oder Levomogetrel in Kombination mit Estrogenen, wie beispielsweise Ethinylestradiol, 17ß-estradiol oder Estradiolvalerat und einem oder mehreren pharmazeutisch annehmbaren Hilfsstoffen/ Trägem realisieren eine laktosefreie orale Kontrazeption. Es wird eine Möglichkeit zur Verbesserung der Prophylaxe der Laktoseintoleranz in einem eventuell beitragenden Faktor gegeben, auch im Hinblick auf aufwendige Untersuchungen zur Laktoseintoleranz. Die Erfindung ist auch zur Langzeitanwendung geeignet.

## Beschreibung

### Technisches Gebiet

Die Erfindung betrifft die Verwendung eines Gestagens in Kombination mit einem Estrogen und einem oder mehreren pharmazeutisch annehmbaren Hilfsstoffen/ Trägern zur Herstellung eines einphasischen pharmazeutischen Präparates zur laktosefreien oralen Kontrazeption. Es wird eine Möglichkeit zur Verbesserung der Prophylaxe der Laktoseintoleranz in einem eventuell beitragenden Faktor gegeben, auch im Hinblick auf aufwendige Untersuchungen zur Laktoseintoleranz.

### Stand der Technik

Laktoseintoleranz, bezeichnet auch als Milchzuckerunverträglichkeit, ist eine Erkrankung , die primär bei ca. 10 Mio. Menschen in Deutschland vorkommt. Typische Beschwerdebilder der Laktoseintoleranz sind je nach Schweregrad Bauchschmerzen, Völlegefühl, Blähungen, Übelkeit und Durchfall. Man unterscheidet grundsätzlich in genetisch bedingter Laktoseintoleranz , beispielsweise durch angeborenen Enzymdefekt, oder erwobener Laktoseintoleranz, deren Ursache noch weitgehend unbekannt ist. Zur Diagnose der Laktoseintoleranz muss ein H2-Atemtest bzw. eine Dünndarmbiopsie durchgeführt werden. Laktose, ein Disaccharid und das Kohlehydrat der. Milch, wird mittels Laktase im Dünndarm in die Einzelzucker gespalten, danach in der Mukosazelle aufgenommen und im Blut weitertransportiert. Gelangt nun der Milchzucker auf Grund fehlender Laktase oder deren verminderter Aktivität ungespalten bzw. teilweise ungespalten in den Dickdarm, so wird der Milchzucker hier durch die angesiedelten Bakterien zu Milch- und Essigsäure, Kohlendioxid, Wasserstoff und Methan zersetzt. In Folge entsteht ein osmotischer Druck, welcher eine verstärkte Darmperistaltik mit Durchfall bewirkt. Gleichzeitig führen die entstandenen Gase im Darm zu Blähungen oder Krämpfen. Je nach Schweregrad der Laktoseintoleranz wird den Betroffenen eine laktosefreie bzw. laktosearme Ernährung (< 3mg Laktose/Tag) angeraten. Der gesunde Erwachsene nimmt täglich 20 bis 30 g Laktose in einer vollwertigen Nahrung auf. 100 g Kuhmilch entsprechen ca. 5 g Laktose. Auch verschiedene Brotsorten und Backwaren, Wurstsorten, Butter und Magarine, Schokolade und Süßstofftabletten enthalten technologisch bedingt Laktose.

Therapieempfehlungen sind auch laktasehaltige Enzympräparate, welche gleichzeitig mit der Nahrungsaufnahme einzunehmen sind, um die Milchzuckerspaltung anzuregen.

US 6,881,428 offenbart die Herstellung laktosefreier Milch, wobei der Milch ein Enzym (Laktase) zugesetzt wird.

Aus der pharmazeutischen Technologie ist bekannt, orale Kontrazeptiva (OC) ausschließlich auf Laktosebasis herzustellen. Laktose als Füllstoff in den Formulierungen besitzt aufgrund seiner herausragenden Eigenschaften besondere Eignung. Die entstehenden Formulierungen zeichnen sich durch Festigkeit, guten Zerfall und gute Stabilität aus. Die Steroidhormone, einzeln oder in Kombination sind in einem Laktosegranulat sehr günstig auf die einzelnen Korngrößenklassen verteilt. Entmischungen des Granulats, die sich in einer ungenügenden Gleichverteilung im Gehalt der Wirkstoffe in den Tablettenkernen bemerkbar machen würden, sind hier kaum bekannt. Man kann davon ausgehen, dass bei einem OC auf Laktosebasis auch im sehr niedrigen Dosisbereich (mindestens 15 µg Ethinylestradiol pro Einheit) der Wirkstoff gleichmäßig verteilt vorliegt. Laktose selbst lässt sich gut granulieren und das Granulat problemlos zu Tabletten verarbeiten. Eine Direkttablettierung, die mit spezieller Laktose denkbar wäre, wird bisher aufgrund von Gehaltsungleichförmigkeiten der Wirkstoffe in den einzelnen Formulierungen nicht in Erwägung gezogen und nicht in der Praxis angewendet. Bisher werden OC mit geringer Wirkstoffdosierung durch Granulierung, anschließende Tablettierung und in den meisten Fällen durch einen Umhüllungsprozess hergestellt.

WO 2005030175 offenbart eine Zusammensetzung mit Norethisteronacetat und Estradiol und Cellulosebindern, wobei die als Beispiel ausgewiesene pharmazeutische Zusammensetzung zu 45 % Laktose enthält.

WO 2005030176 offenbart eine Zusammensetzung mit Gestagenen und Cellulosebindern, wobei die als Beispiel ausgewiesene pharmazeutische Zusammensetzung ebenfalls Norethisteronacetat und Estradiol auch zu 45 % Laktose enthält.

### Darstellung der Erfindung

Aufgabe der Erfindung ist es, eine Möglichkeit zu finden, für Patientinnen mit Laktoseintoleranz bzw. für Frauen, welche bisher ihre Laktoseintoleranz noch nicht erkannt haben, ein orales Kontrazeptivum zu realisieren.

Damit soll gleichzeitig eine Möglichkeit zur Verbesserung der Prophylaxe der Laktoseintoleranz in einem eventuell beitragenden Faktor gegeben werden, auch im Hinblick auf aufwendige Untersuchungen zur Laktoseintoleranz.

Es wurde nun gefunden, dass die Aufgabe erfindungsgemäß gelöst wird durch die Verwendung in Kombination mit Estrogenen und einem oder mehreren pharmazeutisch annehmbaren Hilfsstoffen/ Trägern zur Herstellung eines pharmazeutischen Präparates zur laktosefreien oralen Kontrazeption. Vorzugsweise werden als Gestagen Dienogest in der Dosierung von 2.0 mg oder 1.5 mg oder Chlormadinonacetat oder Levonorgestrel in äquivalenter Dosierung und als Estrogen Ethinylestradiol in der Dosierung von 0.030 mg oder 0.020 mg oder 0.015 mg oder Estradiot oder Estradiotvaterat in äquiva- lenter Dosis verwendet. Das Ethinylestradiol kann auch als Clathrat vorlie- gen.

Es sind auch weitere Gestagene in der erfindungsgemäßen Lösung denkbar.

Die Herstellung der erfindungsgemäßen laktosefreien oralen Kontrazeptiva erfolgt auf Cellulosebasis. Die Formulierungen werden durch Granulierung, Tablettierung oder oft durch Überziehen hergestellt.

Eine bisher nicht praktizierte Herstellungsweise für niedrig dosierte OC's ist die Anwendung von Cellulosen als Füllstoff und die Granulierung der Wirkstoffe mit Bindemitteln. Als Bindemittel wird vorzugsweise Hydroxypropylcellulose (HPC) 1 bis 5% bezogen auf die Kernmasse eingesetzt. Aber auch Hypromellose oder Maltodextrin oder Gelatine oder Stärkekleister können als Bindemittel verwendet werden. Dies wurde bisher nicht in Erwägung gezogen, da bei den im Korngrößenspektrum sehr begrenzten Cellulosen vor allem Probleme bei der Wirkstoffverteilung auftraten.

Die Aufgabe der Erfindung wird durch die in den Ansprüchen 1 bis 7 beschriebenen pharmazeutischen Zusammensetzungen gelöst.

Die Aufgabe wurde erfindungsgemäß auch dadurch gelöst, dass durch Auswahl und ggf. Kombination verschiedener Arten mikrokristalliner Cellulose (MCC), die sich in Schüttdichte, Partikelgröße und Feuchtigkeit unterscheiden wie Avicel PH 101 oder Avicel PH 102 oder Avicel PH 112 oder Kombinationen aus MCC und dibasisches Calciumphosphat oder Mannitol bei gleichzeitiger optimaler Wahl des Verhältnisses Bindemittel zur Gesamtmasse der Formulierung (1-5% zu 100%) überraschenderweise eine Gleichverteilung der meist sehr niedrig dosierten Gestagene und Estrogene in den einzelnen Granulatfraktionen und folgend in der Tablette erzielt wurde. Im Fall des besonders niedrig dosierten EE (minimal sind zur Zeit 15 µg pro Tablette möglich) kann der Wirkstoff zur besseren Verteilung als ethanolische Ethinylestradiolllösung während des Granulierprozesses in das Wirbelbett gesprüht werden.

Die Aufgabe wird auch durch das Verfahren zur Herstellung eines einphasischen pharmazeutischen Präparates zur laktosefreien oralen Kontrazeption gelöst, wobei eine Kombination von einem Gestagen und einem Estrogen zu nx21 täglichen Dosiseinheiten mit nachfolgend nach nx21 maximal 7 täglichen einnahmefreien oder placebohaltige Dosiseinheiten verwendet wird und wobei n gleich 2, 3, 4, 5, 6, 7, 8, 9, 10, 11, 12, 13, 14, 15, 16, und 17 ist. Vorteilhafte Ausgestaltungen der Erfindung sind in den Ansprüchen 18 bis 27 aufgezeigt.

### Ausführungsbeispiele

### Beispiel 1

Wirkstoffkombination:
2.0 mg Dienogest (DNG)/ 0.030 mg Ethinylestradiol (EE)

### Beispiel 2

Wirkstoffkombination:
2.0 mg Chlormadinonacetat (CMA)/ 0.030 mg EEI

### Beispiel 3

Wirkstoffkombination:
0.125 mg Levonorgestrel (LNG)/ 0.030 mg EE

### Beispiel 4

1.5 mg DNG/ 0.015 mg EE, davon 0.825 mg DNG und 0.015 mg EE als Filmbestandteil

Die detailierte Zusammensetzung der Ausführungsbeispiele ist in Tabelle 1 beschrieben.

In Tabelle 2 ist der Vergleich verschiedener Celluloserezepturen hinsichtlich Wirkstoffverteilung im Granulat und Tablettenkernen in Abhängigkeit vom Cellulosetyp und Menge Bindemittel beschrieben.

Gleichverteilung der Wirkstoffe wird über die Bestimmung der Wirkstoffgehälter in den einzelnen Siebfraktionen (Feinkorn, Mittelkorn, Grobkorn) und über die Bestimmung des CUT der Tablettenkerne über den Tablettierprozess (Anfang, Mitte, Ende) angegeben.

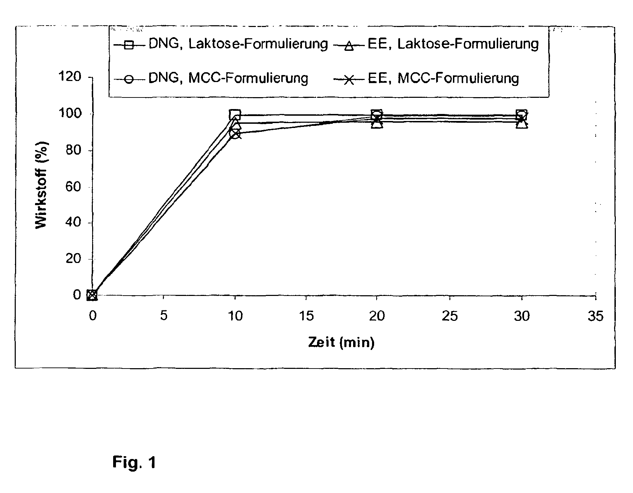

Figur 1 zeigt die Freisetzungsdiagramme der cellulosehaltigen Kombination (2.0 mg DNG und 0.030 mg EE) im Vergleich zur laktosehaltigen Kombination (2.0 mg DNG und 0.030 mg EE), wie mit dem Dissolutiontest in der Freisetzungsapparatur: Paddle unter Verwendung von Wasser mit 37 °C als Dissolutionmedium und 100 U/Min als Rührgeschwindigkeit bestimmt. Es ist ersichtlich, dass die laktosefreie, cellulosehaltige Wirkstoffkombination in gleicher Art freisetzt wie die laktosehaltige Wirkstoffkombination.

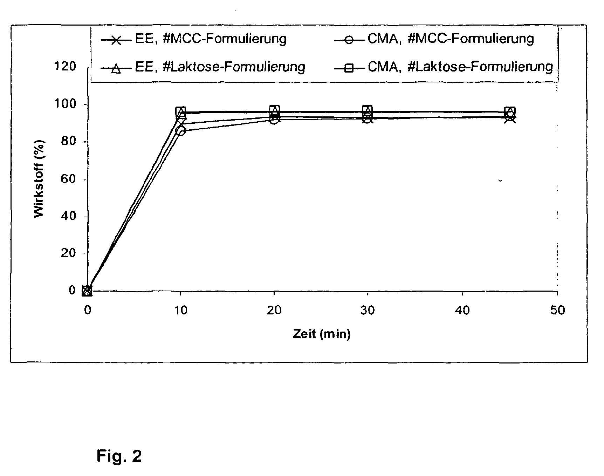

Figur 2 zeigt die Freisetzungsdiagramme der cellulosehaltigen Kombination (2.0 mg CMA und 0.030 mg EE) im Vergleich zur laktosehaltigen Kombination (2.0 mg CMA und 0.030 mg EE), wie mit dem Dissolutiontest in der Freisetzungsapparatur: Paddle unter Verwendung von Natriumdodecylsulfat mit 37 °C als Dissolutionmedium und 75 U/Min als Rührgeschwindigkeit bestimmt. Es ist ersichtlich, dass die laktosefreie, Cellulosehaltigen Wirkstoffkombination in gleicher Art freisetzt wie die laktosehaltigen Wirkstoffkombination.

**Tabelle 1**

| **Formulierung (Kern)** | **1** | **2** | **3** | **4 (MR)*** |
|---|---|---|---|---|
| **Wirkstoff** | 2 mg DNG | 2 mg CMA | 0,125 mg LNG | 0,675 mg DNG |
| | 0,03 mg EE | 0,03 mg EE | 0,03 mg EE | |
| **Füllstoff** | 43,52 mg MCC | 43,52 mg MCC | 45,395 mg MCC | 58,425 mg MCC |
| **Matrixbildner** | --------- | --------- | --------- | 9 mg Hypromellose |
| **Sprengmittel** | 1,95 mg Croscar-mellose Natrium | 1,95 mg Croscar-mellose Natrium | 1,95 mg Croscar-mellose Natrium | 15 mg Maisstärke |
| **Bindemittel** | 2 mg Hydroxy-propylcellulose | 2 mg Hydroxypropylcellulose | 2 mg Hydroxypropylcellulose | 6 mg Maltodextrin |
| **Gleitmittel** | 0,5 mg Magnesiumstearat | 0,5 mg Magnesiumstearat | 0,5 mg Magnesiumstearat | 0,9 mg Magnesiumstearat |

| | | | | |
|---|---|---|---|---|
| *0,825 mg DNG und 0,015 mg E**E** werden zusätzlich als Filmbestandteile in einer Wirkstoffschicht aufgebracht | | | | |

**Tabelle 2**

| | | **Charg.-Nr. 550907** | **Charg.-Nr. 621007** | **Charg.-Nr. 631007** |
|---|---|---|---|---|
| **Zusammensetzung** | | CMA 2 mg | CMA 2 mg | CMA 2 mg |
| | | EE 0,03 mg | EE 0,03 mg | EE 0,03 mg |
| | | Avicel PH 102 44,52 mg | Avicel PH 102 28,52 mg | Avicel PH 101 36,02 mg |
| | | HPC 1 mg Croscarmellose | Avicel PH 112 15 mg | Avicel PH 112 7,5 mg |
| | | Na 1,95 mg | HPC 2 mg | HPC 2 mg |
| | | Mg-sterat 0,5 mg | Croscarmellose Na 1,95 mg | Croscarmellose Na 1,95 mg |
| | | | Mg-sterat 0,5 mq | Mg-sterat 0,5 mg |
| **Verteilung Siebfraktionen** | Feinkorn | CMA 125% | CMA 90,5% | CMA 99,4% |
| | | EE 148 % | EE 104,9 % | EE 107,7 % |
| | Mittelkorn | CMA 92% | CMA 114,9% | CMA 97,3% |
| | | EE 93% | EE 118,5% | EE 96,9% |
| | Grobkorn | CMA 79 % | CMA 125,9 % | CMA 111,9% |
| | | EE 52 % | EE 116 % | EE 102,5 % |
| **CUT Kerne (AV-Wert) Soll: < 15** | Anfang | CMA 2,75 | CMA 3,89 | CMA 2,27 |
| | | EE 3,78 | EE 4,20 | EE 9,65 |
| | Mitte | CMA 4,59 | CMA 7,15 | CMA 3,74 |
| | | EE 5,33 | EE 8,59 | EE 3,01 |
| | Ende | CMA 4,68 | CMA 3,58 | CMA 7,12 |
| | | EE 4,62 | EE 6,71 | EE 9,24 |

## Patentansprüche

1. Pharmazeutische Zusammensetzung zur laktosefreien oralen Kontrazeption enthaltend 2 mg Dienogest in Kombination mit 0.03 mg Ethinylestradiol zusammen mit 58.425 mg mikrokristalliner Cellulose, 9 mg Hypromellose, 15 mg Maisstärke, 6 mg Maltodextrin und 0.9 mg Magnesiumstearat, **dadurch gekennzeichnet, dass** eine Kombination verschiedener Arten mikrokristalliner Cellulosen wie Avicel PH 101, Avicel PH 102 und Avicel PH 112 enthalten ist,
und dass das Gestagen und das Estrogen mit Bindemitteln granuliert wird, wobei keine Laktose enthalten ist.

2. Pharmazeutische Zusammensetzung nach Anspruch 1, **dadurch gekennzeichnet, dass** 0.675 mg Dienogest im Tablettenkern enthalten sind und 0.825 mg DNG sowie 0.015 mg EE zusätzlich als Filmbestandteile in einer Wirkstoffschicht auf den Tablettenkern aufgebracht sind.
